# EUROPÄISCHE PATENTANMELDUNG

(11) **EP 2 500 718 A2**
(43) Veröffentlichungstag der Anmeldung: **19.09.2012**
(21) Anmeldenummer: 12155863.9
(22) Anmeldetag: 17.02.2012
(51) Int. Cl.: G01N 27/06, G01N 33/44

(54) **Verfahren zur Messung der Leitfähigkeitsänderung eines viskoelastischen Materials während seiner Herstellung durch Kombination von rheologischer mit elektrischer Prüfmethode**

(30) Priorität: 14.03.2011 DE 102011001245
(71) Anmelder: ContiTech AG, 30165 Hannover (DE)
(72) Erfinder: Sostmann, Stefan, 30855 Langenhagen (DE); Grönniger, Ivonne, 30659 Hannover (DE); Herrmann, Wolfram, 31515 Wunstorf (DE)
(74) Vertreter: Finger, Karsten

(57) **Zusammenfassung**

Die Erfindung betrifft ein Verfahren zur Messung der Leitfähigkeitsänderung eines viskoelastischen Materials während seiner Herstellung, wobei erfindungsgemäß eine rheologische Prüfmethode zur Erfassung der dynamisch-mechanischen Eigenschaften, wobei sich ein Probekörper des viskoelastischen Materials in der Messkammer eines Rheometers befindet, mit einer elektrischen Prüfmethode zur Erfassung der elektrischen Eigenschaften des viskoelastischen Materials kombiniert wird.

In diesem Zusammenhang wird ein bevorzugter Verfahrensablauf für einen Probekörper aus einem zumindest teilvernetzbaren viskoelastischen Material, insbesondere in Form einer Kautschukmischung, vorgestellt.

## Beschreibung

Die Erfindung betrifft ein Verfahren zur Messung der Leitfähigkeitsänderung eines viskoelastischen Materials während seiner Herstellung.

In der nachveröffentlichten Patentanmeldung DE 10 2009 044 247.2 wird ein Verfahren zur Prüfung der Eigenschaften eines viskoelastischen Materials beschrieben, wobei im Folgenden die Viskoelastizität und ihre bisherige Prüfmethode näher vorgestellt werden.

Die Viskoelastizität ist die Sammelbezeichnung für das viskoelastische Verhalten von Festkörpern, die häufig unter Druckbelastung das Phänomen des Kriechens zeigen. Von besonderer Bedeutung sind dabei Viskopolymere, insbesondere wiederum Viskoelastomere. Diesbezüglich wird beispielsweise auf folgende Literatur verwiesen: Ferry, Viscoelastic Properties of Polymers, S. 33, New York; John Wiley & Sons 1980.

Dynamisch-mechanische Rheometer-Tests sind standardmäßig in der Prüfung viskoelastischer Materialien im Einsatz. Es gibt dabei eine Vielzahl verschiedener Ausführungen dieser Messtechnik, nämlich unterschiedlich bezüglich der Art der Belastung (Druck, Zug, Scherung, Torsion), der Form der Probenhalter und Probenkammer, der aufgebrachten Belastungen und weiterer Bedingungen.

Unter Rheometern werden Rotationsviskometer verstanden, die über erweiterte Steuerungsmöglichkeiten verfügen. Neben der üblichen Steuerung der Drehzahl können die Messungen drehmomentabhängig, winkelabhängig und mit oszillierender Rotation gesteuert werden. Sie erlauben dadurch die sehr genaue Untersuchung spezieller Eigenschaften, beispielsweise der Fließgrenze. Als besonders geeignet haben sich Torsionsrheometer erwiesen, bei denen dynamische Lasten auf eine zwischen zwei Rotoren befindlichen Polymerschicht, insbesondere Elastomerschicht, aufgebracht werden und so dynamisch-mechanische Kennwerte des viskoelastischen Materials gewonnen werden.

Viskoelastische Materialien können elektrisch leitfähig ausgerüstet sein, wobei elektrisch leitfähige Zusätze in diese Materialien eingearbeitet sind. Derartige elektrisch leitfähige Zusätze sind beispielsweise:
- Metalle oder Metallverbindungen
- Ruß in Form von Leitruß oder Standardruß
- Kohlenstoff-Fasern
- Graphit
- Nanotubes, insbesondere aus Kohlenstoff (US 7 338 648)
- Ionische Flüssigkeiten, beispielsweise ein Dialkylimidazoliumchlorid
- Polymere mit elektrisch leitfähigen funktionellen Gruppen, beispielsweise ein Polyethylenglykolester oder Polyethylenglykolcarbonsäureester

Auch Mischungen der vorgenannten Füllstoffgruppen können eingesetzt werden.

Elektrisch leitfähige viskoelastische Materialien werden insbesondere bei der Herstellung folgender Artikel eingesetzt:
- Bei Reifen ist es üblich, ein definiertes Leitfähigkeitsniveau einzustellen, beispielsweise zur Erzeugung antistatischer Eigenschaften in Reifenseitenwänden.
- Bei Fördergurten ist es ebenfalls von Vorteil, wenn die tragseitige und/oder laufseitige Deckplatte, insbesondere die laufseitige Deckplatte, antistatisch ausgerüstet werden.
- Ein besonderer Einsatzschwerpunkt ist der Schlauchsektor. So wird beispielsweise die Außenschicht eines Tankschlauches mit antistatischen Eigenschaften versehen. Ein weiteres Beispiel wird in der Offenlegungsschrift DE 10 2008 014 988 A1 in Form eines mehrschichtigen Schlauches, insbesondere eines Chemikalienschlauches, vorgestellt, der als "Full-Ohm"-Schlauch ausgebildet ist. Die Innenschicht besteht aus einem elektrisch leitfähigen Kunststoff, der beständig gegenüber dem zu fördernden Medium ist. Diese Innenschicht (Inliner) besteht insbesondere aus einer PTFE-Folie. Die zusätzlich vorhandene erste und zweite Zwischenschicht sowie die abriebfeste Außenschicht bestehen jeweils aus einem elektrisch leitfähigen elastomeren Werkstoff.
- In der Offenlegungsschrift DE 10 2007 002 521 A1 wird eine mehrschichtige Stoffbahn mit elektrischer Leitfähigkeit zur Bildung von flexiblen Behältern, Planen, Schutzanzügen, Zelten und dergleichen beschrieben. Neben einer Barriereschicht aus Polyamid, einer Festigkeitsträgerschicht ist auch eine elektrisch leitfähige Elastomerschicht vorhanden, wobei ein Antistatikum in Form eines Weichmachers auf Polymerbasis mit leitfähigen funktionellen Gruppen in die Elastomerschicht eingemischt ist.

Zur elektrischen Charakterisierung von viskoelastischen Materialien gibt es ebenfalls gängige Messtechniken auf dem Markt. Hier gibt es die verschiedensten beschriebenen Ausführungen für Probenhalter und Messaufbauten, die aber allesamt das Material "nur" unter statischen Bedingungen beurteilen.

Ein besonderes Problem liegt nun darin, dass die Leitfähigkeit sehr stark von der Dispersion abhängt und diese mit jedem Schritt in der Herstellkette verändert wird. Daher ist es recht schwer, die Leitfähigkeit im Produkt exakt einzustellen. Da das viskoelastische Material zumeist eine zumindest teilvulkanisierbare Kautschukmischung ist, verändert sich die Leitfähigkeit insbesondere noch einmal bei der Vulkanisation.

Im Rahmen einer Weiterentwicklung besteht nun die Aufgabe der Erfindung darin, ein Verfahren bereitzustellen, das die Messung der Leitfähigkeitsänderung eines viskoelastischen Materials während seiner Herstellung ermöglicht, um eine genaue Vorhersage der Produkteigenschaften von daraus gefertigten Artikeln zu ermöglichen.

Gelöst wird diese Aufgabe dadurch, dass eine rheologische Prüfmethode zur Erfassung der dynamisch-mechanischen Eigenschaften, wobei sich ein Probekörper des viskoelastischen Materials in der Messkammer eines Rheometers befindet, mit einer elektrischen Prüfmethode zur Erfassung der elektrischen Eigenschaften des viskoelastischen Materials kombiniert wird.

Das Prüfen der dynamisch-mechanischen Eigenschaften an viskoelastischen Materialien erlaubt die Vorhersage der Produkteigenschaften von daraus gefertigten Artikeln. Sie ist für die Materialentwicklung ganz entscheidend und zeigt zudem neben den rezepturbezogenen Kennwerten auch ganz empfindlich die "Verarbeitungshistorie" des Materials an. So kann man die Prüfung zur Prozess- und Rezepturkontrolle verwenden. Aus den Messungen erkennt man empfindlich Änderungen in der Zusammensetzung der Inhaltsstoffe, die Qualität der Verteilung der Inhaltsstoffe ineinander und den Zustand (Abbaugrad, Schädigungen usw.) des viskoelastischen Materials.

Durch die Kombination dieses Testkonzeptes mit elektrischen Messungen kann man noch aufgelöster die Eigenschaften des Materials während der dynamischen Belastung verfolgen. Die elektrischen Messungen fokussieren dabei stark auf die Verteilung und die Wechselwirkung der Füllstoffpartikel untereinander und können so die dynamisch-mechanischen Messdaten in ihrer Aussage noch deutlich verbessern.

Dieses kombinierte Messverfahren aus rheologischer und elektrischer Prüfmethode dient nun erfindungsgemäß zur Messung der Leitfähigkeitsänderung eines viskoelastischen Materials während seiner Herstellung.

Besonders vorteilhaft ist es, wenn man im Zusammenhang mit diesem neuen Messverfahren die Messkammer (Rheometerkammer) des Rheometers, der insbesondere als Torsionsrheometer oder dynamischer Rheometertester (DMRT) vorliegt, mit Elektroden ausstattet, die es erlauben, den Widerstand durch den Probekörper und somit dessen elektrischen Eigenschaften zu messen und zu verfolgen. Dabei gibt es verschiedene Möglichkeiten der Elektrodenart und Elektrodenkontaktierung. In der einfachsten Ausführung können die beiden Kammerhälften der Messkammer als Elektroden verwendet werden. Diese sind bauartbedingt voneinander und von dem umgebenden Messaufbau einfach elektrisch zu separieren. Das Rheometer liegt vorzugsweise in Form eines bikonischen Zweiplattensystems vor, wie es beispielsweise im Prüfgerät RPA der Firma Alpha Technologies verwendet wird. Das Rheometer erlaubt es, Scherbedingungen ähnlich den Verarbeitungsschritten während der Herstellung des viskoelastischen Materials auf eine Mischungsprobe wirken zu lassen. Auf diese Weise lassen sich elektrische Leitfähigkeiten vor, nach und sogar während einer Scherbelastung direkt vergleichen. Dies gilt auch während der Vulkanisation einer Kautschukmischung, was im Rahmen eines Ausführungsbeispiels noch näher dargelegt wird.

Die Messung an den bevorzugt eingesetzten bikonischen Kammerplatten erlaubt zwar keine Umrechnung der gemessenen Leitfähigkeiten in spezifische Widerstände oder spezifische Leitwerte. Trotzdem ist der relative Vergleich innerhalb der Proben gewährleistet und ein Vergleich der prozessschrittabhängigen Messungen zu genormten DIN-Leitfähigkeitsmessungen gegeben.

In der geschlossenen und unter Schließdruck gehaltenen Messkammer des Rheometers wird zur Erfassung der Leitfähigkeitsänderung wenigstens eine Anfangsleitfähigkeit und eine Endleitfähigkeit des Probekörpers gemessen, verbunden mit einer Bearbeitung des viskoelastischen Materials. Die Bearbeitung hängt dabei von der Art des viskoelastischen Materials ab. Bei der Herstellung von Elastomeren und thermoplastischen Elastomeren ist es die Vernetzung (Vollvernetzung, Teilvernetzung) durch Vulkanisation. Bei Thermoplasten wird häufig eine Wärmebehandlung durchgeführt.

Außer der Messung der Anfangsleitfähigkeit und der Endleitfähigkeit können während der Bearbeitungsphase des viskoelastischen Materials weitere Leitfähigkeitsmessungen durchgeführt werden, verbunden mit einer Diagrammauswertung.

Der Probekörper besteht insbesondere aus einem zumindest teilvernetzbaren viskoelastischen Material, wobei im Folgenden ein vorteilhafter Verfahrensablauf in wenigstens vier Verfahrensschritten näher erläutert wird:
- Nach einem ersten Verfahrensschritt wird ein Probekörper aus einem zumindest teilvernetzbaren viskoelastischen Materials in die Messkammer des Rheometers eingelegt. Der Probekörper hat eine Volumenmasse von wenigstens 3 cm³, insbesondere wenigstens 5 cm³, und liegt dabei beispielsweise plättchenförmig vor. In der Messkammer herrscht zumeist eine Temperatur von wenigstens120°C vor, und zwar bei einem bevorzugten Temperaturbereich von 120 bis 180°C.
- Nach einem zweiten Verfahrensschritt wird nach einer Ruhephase von wenigstens 30 Sekunden die elektrische Leitfähigkeit des Probekörpers als Anfangsleitfähigkeit gemessen.
- Nach einem dritten Verfahrensschritt wird der Probekörper unter den üblichen Rheometerbedingungen zumindest teilvernetzt, und zwar insbesondere bei einem Temperaturbereich von insbesondere 150°C und 180°C.
- Nach einem vierten Verfahrensschritt wird nach dem Abkühlen des Probekörpers erneut die elektrische Leitfähigkeit des zumindest teilvernetzten Probekörpers als Endleitfähigkeit gemessen. Die Abkühlung des Probekörpers wird insbesondere auf mindestens 70°C durchgeführt.

Das neue Verfahren kann auf jeden Prozessschritt in der Fertigungskette (z.B. Mischen, Nacharbeiten auf dem Walzwerk, Extrudieren, Kalandrieren) angewendet werden und zeigt direkt den Einfluss auf die Leitfähigkeitseigenschaften des viskoelastischen Materials.

Das neue Verfahren wird insbesondere zur Messung der Leitfähigkeitsänderung einer Kautschukmischung, enthaltend wenigstens eine Kautschukkomponente, einen elektrisch leitfähigen Zusatz und weitere Mischungsingredienzien, eingesetzt. Als Kautschukkomponenten sind insbesondere zu nennen:
Ethylen-Propylen-Kautschuk (EPM)
Ethylen-Propylen-Dien-Kautschuk (EPDM)
Nitrilkautschuk (NBR)
(teil)hydrierter Nitrilkautschuk (HNBR)
Fluor-Kautschuk (FKM)
Chloropren-Kautschuk (CR)
Naturkautschuk (NR)
Styrol-Butadien-Kautschuk (SBR)
Isopren-Kautschuk (IR)
Butylkautschuk (IIR)
Brombutylkautschuk (BIIR)
Chlorbutylkautschuk (CIIR)
Bromiertes Copolymer aus Isobutylen und Paramethylstyrol (BIMS)
Butadien-Kautschuk (BR)
Chloriertes Polyethylen (CM)
Chlorsulfoniertes Polyethylen (CSM)
Polyepichlorhydrin (ECO)
Terpolymere des ECO mit Ethylenoxid und ungesättigten Monomeren (ETER)
Ethylen-Vinylacetat-Kautschuk (EVA)
Acrylat-Kautschuk (ACM)
Ethylen-Acrylat-Kautschuk (AEM)
Silikonkautschuk (MQ, VMQ, PVMQ, FVMQ; DE 10 2006 058 470 A1)
Fluorierter Methylsilikonkautschuk (MFQ)
Perfluorinierter Propylen-Kautschuk (FFPM)
Perfluorcarbon-Kautschuk (FFKM)
Polyurethan (PU)

Die vorgenannten Kautschuktypen können unverschnitten sein. Auch der Einsatz eines Verschnittes, insbesondere in Verbindung mit einem der vorgenannten Kautschuktypen, beispielsweise ein NR/BR-Verschnitt oder ein BR/SBR-Verschnitt, ist möglich.

Von besonderer Bedeutung sind folgende Kautschuktypen mit vielfältigem Einsatz in der Kautschuktechnologie: ACM, AEM, BIMS, CM, CR, IIR, BIIR, CIIR, ECO, EPM, EPDM, ETER, EVA, FKM, HNBR, NBR, NR, SBR, VMQ und FVMQ.

Die Kautschukmischung enthält einen elektrisch leitfähigen Zusatz, insbesondere in Form von Ruß, wobei der Ruß zudem als Füllstoff wirkt.

Die weiteren Mischungsingredienzien umfassen wenigstens einen Vernetzer oder ein Vernetzersystem (Vernetzungsmittel und Beschleuniger). Weitere Mischungsingredienzien sind zumeist noch ein Verarbeitungshilfsmittel und/oder ein Weichmacher und/oder ein Alterungsschutzmittel sowie gegebenenfalls weitere Zusatzstoffe (z.B. Farbpigmente, Fasern). Auch nichtleitfähige Füllstoffe, beispielsweise Kieselsäure und/oder Silikate können noch eingemischt sein. Diesbezüglich wird auf den allgemeinen Stand der Kautschukmischungstechnologie verwiesen.

Unter Vollvernetzung derartiger Kautschukmischungen durch Vulkanisation werden Elastomere gebildet.

Auch thermoplastische Kautschukmischungen, verbunden mit einer Teilvernetzung und Ausbildung von thermoplastischen Vulkanisaten - auch thermoplastische Elastomere mit der Kurzbezeichnung TPE - genannt, können hiermit untersucht werden. Derartige Mischungen enthalten wenigstens eine Kautschukkomponente, wenigstens eine Thermoplastkomponente, einen elektrisch leitfähigen Zusatz und weitere Mischungsingredienzien.

Die bevorzugten Kautschukkomponenten sind hier: ACM, AEM, CR, EPM, EPDM, FKM, NBR, NR und SBR. Diese Kautschukkomponenten sind insbesondere mit keiner weiteren Kautschukkomponente verschnitten.

Die bevorzugten Thermoplastkomponenten sind ein:
Polyolefin, insbesondere Polyethylen (PE) oder Polypropylen (PP)
Polystyrol
Polyamid (PA), beispielsweise PA6 oder PA6.6
Polyester, beispielsweise PET, PEN oder PBT

Was den elektrisch leitfähigen Zusatz und die weiteren Mischungsingredienzien betrifft, so wird auf das bereits Erwähnte verwiesen.

Eine bevorzugte thermoplastische Kautschukmischung wird insbesondere in der Offenlegungsschrift DE 100 04 632 A1 beschrieben.

Weiterhin können mit dem neuen Verfahren auch thermoplastische Kunststoffe, in die elektrisch leitfähige Zusätze sowie gegebenenfalls weitere Mischungsingredienzien eingearbeitet sind, untersucht werden. Außer den bereits erwähnten Werkstofftypen im Zusammenhang mit den Thermoplastkomponenten bei der Herstellung von thermoplastischen Elastomeren sind hier insbesondere noch die Fluorkunststoffe zu nennen, insbesondere wiederum folgende Typen:
- Polytetrafluorethylen (PTFE)
- Modifiziertes Polytetrafluorethylen (TFM)
- Fluorethylenpolymer (FEP)
- Perfluoralkylvinylether-Tetrtaethylen-Copolymer (PFA)
- Ethylen-Tetrafluorethylen-Copolymer (ETFE)
- Polyvinylfluorid (PVF)
- Polyvinylidenfluorid (PVDF)

Von herausragender Bedeutung ist dabei elektrisch leitfähiges PTFE als Innenschicht von Schläuchen, insbesondere Chemikalienschläuchen.

Da das elektrisch leitfähige PTFE nach der Offenlegungsschrift DE 10 2008 014 988 A1 in Schläuchen insbesondere in Form einer Folie oder eines nahtlos extrudierten Innenschlauches vorliegt, verbunden mit einer diesbezüglichen Herstellung unter dem Einfluss von Wärme, wird der Probekörper wärmebehandelt, beispielsweise unter Extrusionsbedingungen.

Der Schwerpunkt des erfindungsgemäßen Verfahrens liegt jedoch auf der Untersuchung von thermoplastfreien Kautschukmischungen, die nach der Vulkanisation vollvernetzte Elastomere bilden.

Die aus diesen viskoelastischen Materialien hergestellten Artikel mit elektrischer Leitfähigkeit können für folgende Artikeltypen verwendet werden:
- Schläuche (z.B. Kraftfahrzeugschlauch, Schwimmschlauch);
- Luftfederbälge (Axialbalg, Kreuzlagenbalg);
- Kompensatoren (z.B. Lateralkompesator, Torsionskompensator);
- Antriebsriemen (Flachriemen, Keilriemen, Keilrippenriemen, Zahnriemen);
- Fördergurte (Textilgurt, Stahlseilgurt);
- Reifen;
- Gummierte Stoffe (z.B. Behälterstoff, Bootsstoff, Schutzanzugstoff);
- Innenraumverkleidungen;
- Buchsen und Lager;
- Dichtungen in Form eines Extrudates oder eines Formartikels.

Von besonderer Bedeutung sind Reifen, Fördergurte, Schläuche und mehrschichtige Stoffbahnen.

Die Erfindung wird nun anhand eines Ausführungsbeispiels unter Bezugnahme auf ein Diagramm näher erläutert, und zwar bei folgenden Versuchsdaten:
- Probekörper
Der Probekörper hatte bei einer Volumenmasse von 5 cm³ in Plättchenform folgende Zusammensetzung in phr (per hundred rubber):

| | | |
|---|---|---|
| NR TSR-L | 100 | Standardisierter malayischer NR; DE 10 2008 008 105 A1 |
| Ruß N339 | 50 | elektrisch leitfähiger Ruß |
| Zinkoxid | 5 | |
| Stearinsäure | 1 | |
| 6PPD | 5 | N-(1,3-Dimethylbutyl)-N'-phenyl-p-phenylendiamin als Alterungsschutzmittel |
| Schwefel | 0,8 | Vernetzungsmittel |
| CBS | 2,5 | N-Cyclohexyl-2-benzothiazylsulfenand als Beschleuniger |

Die mit dem elektrisch leitfähigen Ruß gefüllte Kautschukmischung wurde im Rahmen eines Standardmischverfahrens auf einem Laborinnenmischer mit ineinandergreifender
Rotorgeometrie hergestellt.
- Prüfgerät (Messtechnik)
Rheometer DMRT: RPA der Firma Alpha Technologies
- Verfahrensablauf (Messablauf)

| | |
|---|---|
| Erster Verfahrensschritt: | Der Probekörper wurde in die Messkammer des Rheometers bei 160°C eingelegt. |
| Zweiter Verfahrensschritt: | Nach 30 Sekunden Ruhephase wurde die elektrische Leitfähigkeit gemessen (Anfangsleitfähigkeit). |
| Dritter Verfahrensschritt: | Der Probekörper wurde unter üblichen Rheometerbedingungen vernetzt, und zwar bei 160°C, 0,5° Auslenkung, 1.667 Hz. |
| Vierter Verfahrensschritt: | Nach Abkühlen auf 70°C wurde erneut die elektrische Leitfähigkeit gemessen (Endleitfähigkeit). |

- Diagramm

| | |
|---|---|
| Ordinate: | Widerstand [Ohm] |
| Abszisse: | Zeit [Sekunden] |

Das Diagramm veranschaulicht den Verlauf der Leitfähigkeit während des Experimentes mit mehreren Leitfähigkeitsmessungen während der Bearbeitung der Kautschukmischung, verbunden mit folgenden wesentlichen Daten:

| | |
|---|---|
| Anfangsleitfähigkeit: | 4,2 kOhm |
| Endleitfähigkeit: | 12 Ohm |

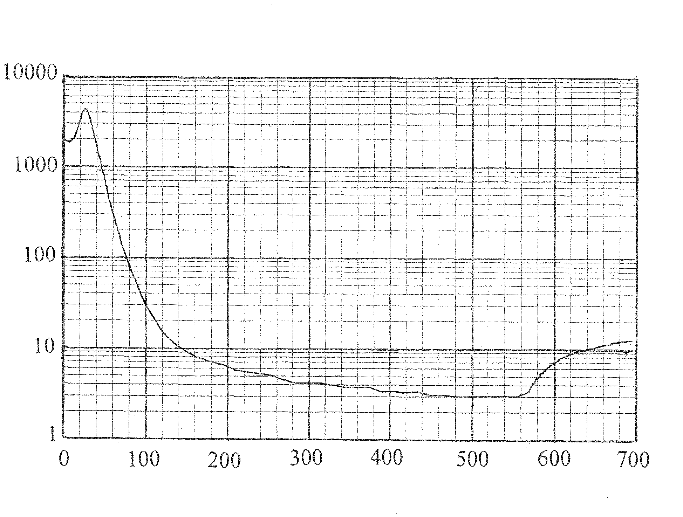

## Patentansprüche

1. Verfahren zur Messung der Leitfähigkeitsänderung eines viskoelastischen Materials während seiner Herstellung, **dadurch gekennzeichnet, dass** eine rheologische Prüfmethode zur Erfassung der dynamisch-mechanischen Eigenschaften, wobei sich ein Probekörper des viskoelastischen Materials in der Messkammer eines Rheometers befindet, mit einer elektrischen Prüfmethode zur Erfassung der elektrischen Eigenschaften des viskoelastischen Materials kombiniert wird.

2. Verfahren nach Anspruch 1, **dadurch gekennzeichnet, dass** im Rahmen der rheologischen Prüfmethode die Messkammer des Rheometers mit Elektroden ausgestattet wird, die es erlauben, den Widerstand durch den Probekörper zu messen und zu verfolgen.

3. Verfahren nach Anspruch 2, **dadurch gekennzeichnet, dass** als Elektroden ein bikonisches Zweiplattensystem eingesetzt wird.

4. Verfahren nach einem der Ansprüche 1 bis 3, **dadurch gekennzeichnet, dass** ein Probekörper mit einer Volumenmasse von wenigstens 3 cm³ eingesetzt wird.

5. Verfahren nach einem der Ansprüche 1 bis 4, **dadurch gekennzeichnet, dass** in der geschlossenen und unter Schließdruck gehaltenen Messkammer des Rheometers zur Erfassung der Leitfähigkeitsänderung wenigstens eine Anfangsleitfähigkeit und Endleitfähigkeit des Probekörpers gemessen wird, verbunden mit einer Bearbeitung des viskoelastischen Materials.

6. Verfahren nach Anspruch 5, **dadurch gekennzeichnet, dass**
- nach einem ersten Verfahrensschritt ein Probekörper aus einem zumindest teilvernetzbaren viskoelastischen Materials in die Messkammer des Rheometers eingelegt wird;
- nach einem zweiten Verfahrensschritt nach einer Ruhephase die elektrische Leitfähigkeit des Probekörpers als Anfangsleitfähigkeit gemessen wird;
- nach einem dritten Verfahrensschritt der Probekörper unter Rheometerbedingungen zumindest teilvernetzt wird;
- nach einem vierten Verfahrensschritt nach dem Abkühlen des Probekörpers in der geschlossenen und unter Schließdruck gehaltenen Messkammer erneut die elektrische Leitfähigkeit des zumindest teilvernetzten Probekörpers als Endleitfähigkeit gemessen wird.

7. Verfahren nach Anspruch 6, **dadurch gekennzeichnet, dass** nach dem ersten Verfahrensschritt der Probekörper bei wenigstens 120°C in die Messkammer des Rheometers eingelegt wird.

8. Verfahren nach Anspruch 7, **dadurch gekennzeichnet, dass** in der Messkammer des Rheometers eine Temperatur von 120°C bis 180°C vorherrscht.

9. Verfahren nach einem der Ansprüche 6 bis 8, **dadurch gekennzeichnet, dass** nach dem dritten Verfahrensschritt die Vernetzung bei einer Temperatur von 150°C bis 180°C durchgeführt wird.

10. Verfahren nach einem der Ansprüche 6 bis 9, **dadurch gekennzeichnet, dass** nach dem vierten Verfahrensschritt der Probekörper auf mindestens 70°C abgekühlt wird.

11. Verfahren nach einem der Ansprüche 6 bis 10, **dadurch gekennzeichnet, dass** als viskoelastisches Material eine Kautschukmischung, enthaltend wenigstens eine Kautschukkomponente, einen leitfähigen Zusatz und weitere Mischungsingredienzien, zur Messung der Leitfähigkeitsänderung eingesetzt wird.

12. Verfahrens nach Anspruch 11, **dadurch gekennzeichnet, dass** als Kautschukkomponente Ethylen-Propylen-Kautschuk (EPM), Ethylen-Propylen-Dien-Kautschuk (EPDM), Nitrilkautschuk (NBR), (teil)hydrierter Nitrilkautschuk (HNBR), Fluor-Kautschuk (FKM), Chloropren-Kautschuk (CR), Naturkautschuk (NR), Styrol-Butadien-Kautschuk (SBR), Isopren-Kautschuk (IR), Butylkautschuk (IIR), Brombutylkautschuk (BIIR), Chlorbutylkautschuk (CIIR), Bromiertes Copolymer aus Isobutylen und Paramethylstyrol (BIMS), Butadien-Kautschuk (BR), Chloriertes Polyethylen (CM), Chlorsulfoniertes Polyethylen (CSM), Polyepichlorhydrin (ECO), Terpolymere des ECO mit Ethylenoxid und ungesättigten Monomeren (ETER), Ethylen-Vinylacetat-Kautschuk (EVA), Acrylat-Kautschuk (ACM), Ethylen-Acrylat-Kautschuk (AEM), Silikonkautschuk (MQ, VMQ, PVMQ, FVMQ), Fluorierter Methylsilikonkautschuk (MFQ), Perfluorinierter Propylen-Kautschuk (FFPM), Perfluorcarbon-Kautschuk (FFKM) oder Polyurethan (PU) eingesetzt wird, wobei die vorgenannten Kautschuktypen verschnittfrei oder in einem Verschnitt, insbesondere in Verbindung mit einem der vorgenannten Kautschuktypen, zum Einsatz gelangen.
